(19) Europäisches Patentamt
European Patent Office
Office européen des brevets

(11) Veröffentlichungsnummer: **0 088 986**
**B1**

# (12) EUROPÄISCHE PATENTSCHRIFT

(45) Veröffentlichungstag der Patentschrift: **25.02.87**

(51) Int. Cl.⁴: **C 07 D 401/12**, C 07 D 405/12, A 61 K 31/34, A 61 K 31/40

(21) Anmeldenummer: **83102247.0**

(22) Anmeldetag: **08.03.83**

## (54) Bicyclische Phenolether, Verfahren zu deren Herstellung sowie diese Verbindungen enthaltende Arzneimittel.

(30) Priorität: **13.03.82 DE 3209271**

(43) Veröffentlichungstag der Anmeldung: **21.09.83 Patentblatt 83/38**

(45) Bekanntmachung des Hinweises auf die Patenterteilung: **25.02.87 Patentblatt 87/9**

(84) Benannte Vertragsstaaten: **AT BE CH DE FR GB IT LI LU NL SE**

(56) Entgegenhaltungen:
**EP-A-0 013 894**
**EP-A-0 014 928**
**EP-A-0 026 876**

**JOURNAL OF MEDICINAL CHEMISTRY, Band 17, Nr. 7, Juli 1974, Seiten 739-744, Columbus, Ohio, USA J.L. ARCHIBALD et al.: "Benzamidopiperidines. 3. Carbocyclic derivatives related to indoramin"**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH, Sandhofer Strasse 116, D-6800 Mannheim 31 (DE)**

(72) Erfinder: **Friebe, Walter- Gunar, Dr., Heidelberger Landstrasse 111d, D-6100 Darmstadt- 13 (DE)**
Erfinder: **Kampe, Wolfgang, Dr., Zedernstrasse 49, D-6805 Heddesheim (DE)**
Erfinder: **Roesch, Androniki, Dr., Werderstrasse 44, D-6800 Mannheim- 1 (DE)**
Erfinder: **Schaumann, Wolfgang, Prof. Dr., Mönchhofstrasse 58, D-6900 Heidelberg (DE)**

**EP 0 088 986 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue bicyclische Phenolether, Verfahren zu deren Herstellung sowie diese Verbindungen enthaltende Arzneimittel.

Die neuen Verbindungen sowie ihre pharmakologisch unbedenklichen Salze wirken oral bereits in niedrigen Dosen hemmend auf anaphylaktische Reaktionen der Haut und des Bronchialsystems. Ein schwacher antioedematoeser Effekt wurde beobachtet. Gleichzeitig antagonisieren sie auch durch Allergen freigesetzte Mediatoren, z.B. Histamin.

Verbindungen aehnlicher Konstitution sind in der DE-A 29 01 336 beansprucht. Es wurde nun gefunden, daß auch Verbindungen, die an Stelle des Cumarinrestes einen Benzofuran-, Benzothiophenoder Benzopyrrolrest tragen, eine ausgepraegte antiallergische Wirkung zeigen.

Die Erfindung betrifft bicyclische Phenolether der allgemeinen Formel I

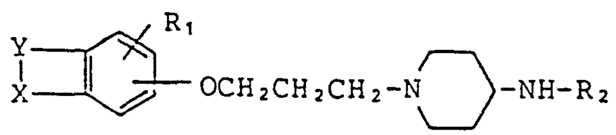
(I),

in welcher

$R_1$ Wasserstoff oder einen $C_1$-$C_6$-Alkylrest,

$R_2$ Wasserstoff, eine Phenacetyl-, $C_3$-$C_7$-Cycloalkyl-carbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkylsulfinyl, $C_1$-$C_6$-Alkylsulfonyl oder Cyano substituiert sein kann,

X ein Sauerstoffatom oder eine NH-Gruppe,

Y eine Gruppe -CO-$CHR_3$-, eine Gruppe $-CO-C=CR_4R_5$ oder $R_4R_5C=C-CO-$,

$R_3$ Wasserstoff, einen $C_1$-$C_6$-Alkylrest, der gegebenenfalls durch Phenyl substituiert sein kann oder einen $C_3$-$C_7$-Cycloalkylrest,

$R_4$ Wasserstoff oder einen $C_1$-$C_6$-Alkylrest und

$R_5$ Wasserstoff, einen $C_1$-$C_6$-Alkylrest, einen Phenyl- oder Styryl- oder einen Farnesyl-, Geranyl- und (2,6,6-Tri-methyl-1-cyclohexen-1-yl)-vinylrest bedeuten, wobei $R_4$ und $R_5$ zusammen mit dem C-Atom auch einen $C_3$-$C_7$-Cyclo-alkylring bilden können,

sowie deren Salze mit pharmakologisch verträglichen Säuren.

Weiterhin betrifft die Erfindung pharmazeutische Praeparate mit einem Gehalt an Verbindungen der allgemeinen Formel I sowie die Verwendung von Verbindungen der allgemeinen Formel I zur Herstellung solcher Praeparate.

Die fuer $R_1$, $R_3$, $R_4$ und $R_5$ stehenden niederen Alkylreste weisen 1-6, insbesondere 1-4 Kohlenstoffatome auf und koennen geradkettig oder verzweigt sein. Bevorzugt ist der Methyl-, Isopropyl- und Propylrest.

Die Grundkörper, die X und Y mit dem Phenylteil bilden können, sind Cumaran-2-on, Cumaran-3-on, Oxindol und Thianaphthen-3-on.

Die Oxypropylgruppe steht in der Regel in der 4, 5-oder 6-Stel-lung des jeweiligen Ringsystems.

Außer den in den Beispielen genannten Verbindungen sind Gegenstand der Erfindung insbesondere alle Substanzen, die jede moegliche Kombination der in den Beispielen genannten Substituenten aufweisen.

Das erfindungsgemaeße Verfahren zur Herstellung der Verbindungen der allgemeinen Formel I ist dadurch gekennzeichnet, daß man in an sich bekannter Weise eine Verbindung der allgemeinen Formel II

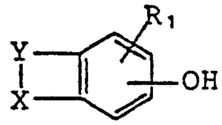
(II),

in der

$R_1$, X und Y die obengenannte Bedeutung haben, mit einer Verbindung der allgemeinen Formel III

L-$CH_2CH_2CH_2$-M (III),

in der

L und M reaktive Reste bedeuten,

und einer Verbindung der allgemeinen Formel IV

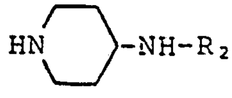
(IV),

in der
$R_2$ die obengenannte Bedeutung hat,
umsetzt,
anschließend gewuenschtenfalls die Gruppe $R_2$ durch bekannte Verfahren in eine andere Gruppe $R_2$ umwandelt,
fuer den Fall, daß $R_3$ ein Wasserstoffatom bedeutet, gegebenenfalls mit einer Verbindung $R_4COR_5$ oder einem reaktiven Derivat davon kondensiert,
fuer den Fall, daß Y die Gruppe $-CO-\underset{|}{C}=CR_4R_5$ bedeutet,
gewuenschtenfalls die Doppelbindung reduziert,
und das so erhaltene Reaktionsprodukt gewuenschtenfalls in ein pharmakologisch vertraegliches Salz ueberfuehrt.

Als reaktive Reste L und M der Verbindungen der allgemeinen Formel III kommen Chlor, Brom, Mesyloxy und Tosyloxy in Frage.

Das erfindungsgemaeße Verfahren fuehrt man beispielsweise so durch, daß man zunaechst eine Verbindung der allgemeinen Formel III mit einer Verbindung der allgemeinen Formel IV kondensiert und das erhaltene Reaktionsprodukt isoliert. Dieses Zwischenprodukt wird dann mit einer Verbindung der allgemeinen Formel II zur Reaktion gebracht. Die Umsetzung erfolgt zweckmaeßig in alkalischem Medium, beispielsweise in einem niederen Alkohol wie z.B. Isopropanol in Anwesenheit von Natriumisopropanolat oder in Dimethylformamid oder Tetrahydrofuran in Gegenwart von Kaliumcarbonat oder Triethylamin.

Eine andere Variante besteht darin, zunaechst eine Verbindung der allgemeinen Formel II mit einer Verbindung der allgemeinen Formel III zur Reaktion zu bringen; anschließend wird das erhaltene Reaktionsprodukt mit einer Verbindung der allgemeinen Formel IV zum gewuenschten Endprodukt der allgemeinen Formel I umgesetzt.

Eine nachtraegliche Umwandlung der Gruppe $R_2$ in der allgemeinen Formel I in eine andere Gruppe $R_2$ erfolgt beispielsweise durch Acylierung einer Verbindung der allgemeinen Formel I, in der $R_2$ fuer Wasserstoff steht, mit einer Verbindung $R_2$-Z, wobei Z einen reaktiven Rest darstellt. Verbindungen der allgemeinen Formel I mit der Bedeutung $R_2$ = Wasserstoff stellen somit wertvolle Zwischenprodukte zur Herstellung anderer Verbindungen der allgemeinen Formel I dar. Reaktive Reste Z koennen alle Reste sein, die in der Peptidchemie zur Aktivierung von Carbonsaeuren Verwendung finden, beispielsweise Halogenatome, die Azidogruppe, Alkyloxy-, Aryloxy- und Acyloxy-Gruppen.

Eine weitere Moeglichkeit der nachtraeglichen Umwandlung der Gruppe $R_2$ in der allgemeinen Formel I besteht darin, daß man nach allgemein bekannten Verfahren einen oder mehrere Substituenten des Acylrestes $R_2$ beispielsweise durch Veresterung, Verseifung, Reduktion, Alkylierung, Acylierung, Hydrogenolyse, Oxidation, Amidierung oder Eliminierung in einen oder mehrere andere Substituenten des Acylrestes $R_2$ umwandelt.

Eine gewuenschtenfalls vorzunehmende Kondensation einer Verbindung der allgemeinen Formel I, in der $R_3$ ein Wasserstoffatom bedeutet, mit einer Carbonylverbindung $R_4COR_5$ oder einem reaktiven Derivat hiervon erfolgt zweckmaeßig in einem organischen Loesungsmittel in Gegenwart eines wasserabspaltenden Mittels, beispielsweise in einem niederen Alkohol oder in Toluol in Gegenwart von Kaliumhydroxid oder Ammoniak oder Toluolsulfonsaeure.

Die Reduktion einer fuer Y stehenden Gruppe $-CO-\underset{|}{C}=CR_4R_5$ kann beispielsweise mit katalytisch erregtem Wasserstoff in Anwesenheit eines Edelmetallkatalysators wie Palladium oder Platin erfolgen.

Die Ausgangsverbindungen der allgemeinen Formel II, III und IV sind literaturbekannte Substanzen oder koennen in Analogie zu literaturbekannten Verfahren hergestellt werden.

Die pharmakologisch vertraeglichen Salze erhaelt man in ueblicher Weise, z.B. durch Neutralisation der Verbindungen der allgemeinen Formel I mit nichttoxischen anorganischen oder organischen Saeuren wie z.B. Salzsaeure, Schwefelsaeure, Phosphorsaeure, Bromwasserstoffsaeure, Essigsaeure, Milchsaeure, Citronensaeure, Aepfelsaeure, Salicylsaeure, Malonsaeure, Maleinsaeure oder Bernsteinsaeure.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die Substanzen der allgemeinen Formel I koennen in fluessiger oder fester Form oral und parenteral appliziert werden. Als Injektionsmedium kommt vorzugsweise Wasser zur Anwendung, welches die bei Injektionsloesungen ueblichen Stabilisierungsmittel, Loesungsvermittler und / oder Puffer enthaelt. Derartige Zusaetze sind z.B. Tartrat- oder Borat-Puffer, Ethanol, Dimethylsulfoxid Komplexbildner (wie Ethylendiamintetraessigsaeure), hochmolekulare Polymere (wie fluessiges Polyethylenoxid) zur Viskositaetsregulierung oder Polyethylen-Derivate von Sorbitanhydriden.

Feste Traegerstoffe sind z.B. Staerke, Lactose, Mannit, Methylcellulose, Talkum, hochdisperse Kieselsaeure, hoehermolekulare Polymere (wie Polyethylenglykole).

Fuer die orale Applikation geeignete Zubereitungen koennen gewuenschtenfalls Geschmacks-und Sueßstoffe enthalten. Fuer die aeußerliche Anwendung koennen die erfindungsgemaeßen Substanzen I auch

in Form von Pudern und Salben verwendet werden. Sie werden dazu z.B. mit pulverfoermigen, physiologisch vertraeglichen Verduennungsmittel bzw. ueblichen Salbengrundlagen vermischt.

Die verabreichte Dosierung haengt vom Alter, der Gesundheit und dem Gewicht des Empfaengers, dem Ausmaß der Krankheit, der Art gleichzeitiger gegebenenfalls durchgefuehrter weiterer Behandlungen, der Haeufigkeit der Behandlungen und der Art der gewuenschten Wirkung ab. Ueblicherweise betraegt die taegliche Dosis der aktiven Verbindung 0.1 bis 50 mg/kg Koerpergewicht. Normalerweise sind 0.5 bis 40 und vorzugsweise 1.0 bis 20 mg/ kg/Tag in einer oder mehreren Anwendungen pro Tag wirksam, um die gewuenschten Resultate zu erhalten.

Außer den in den nachstehenden Beispielen genannten Substanzen sind im Sinne der Erfindung die folgenden Verbindungen bevorzugt:

6-[3-(4-Benzamido-piperidino)propoxy]-2-isopropyliden-benzo[b]thio-phen-3-on

6-[3-(4-Phenylacetamido-piperidino)propoxy]-2-isopropyliden-benzo[b]thiophen-3-on

6-<3-[4-(2-Nitro-benzamido)piperidino)propoxy>-2-isopropyliden-benzo[b]thiophen-3-on

**Beispiel 1**

**6-[3-(4-Benzamido-piperidino)propoxy]-2-cyclohexyliden-cumaranon-3**

Zu der Loesung von 0.58 g (25 mmol) Natrium in 75 ml 2-Pro-panol gibt man bei 40°C 6.1 g (25 mmol) 2-Cyclohexyliden-6-hydroxy-cumaranon-3, ruehrt 10 min bei 40°C, fuegt 8.4 g (30 mmol) 3-(4-Benzamido-piperidino)propylchlorid zu, erhitzt 6 h zum Rueckfluß, kuehlt ab und kristallisiert den Niederschlag aus Ethanol um.

Man isoliert 8.7 g Titelverbindung (75 % d.Th.) vom Schmp. 174 - 175°C.

Das als Ausgangsstoff eingesetzte 2-Cyclohexyliden-6-hydroxy-cumaranon-3 kann wie folgt erhalten werden:

Eine Mischung aus 20.0 g (0.13 mol) 6-Hydroxy-cumaranon-3, 60 ml Cyclohexanon, 600 ml Ethanol und 50 g Kaliumhydroxid wird 36 h bei Raumtemperatur geruehrt, filtriert und das Filtrat mit Salzsaeure angesaeuert.

Es fallen 11.7 g 2-Cyclohexyliden-6-hydroxy-cumaranon-3 (40 % d.Th.) vom Schmp. 210 - 212°C aus.

**Beispiel 2**

In analoger Weise wie in Beispiel 1 beschrieben erhaelt man:

| | Bezeichnung | Ausbeute % | Schmelzpunkt°C (Loesungsmittel) |
|---|---|---|---|
| a) | 5-[3-(4-Benzamido-piperidino)propoxy]-3-isopropyliden-oxindol<br>aus 5-Hydroxy-3-isopropyliden-oxindol und 3-(4-Benzamido-piperidino)propyl-chlorid | 36 | 205 (Diethylether) |
| b) | 5-<3-[4-(4-Fluor-benzamido)piperidino] propoxy>-3-isopropyliden-oxindol<br>aus 5-Hydroxy-3-isopropyliden-oxindol und 3-[4-(4-Fluor-benzamido)piperidino] propylchlorid | 24 | 214 (Diethylether) |
| c) | 5-<3-[4-(3-Methyl-benzamido)piperidino] propoxy>-3-isopropyliden-oxindol<br>aus 5-Hydroxy-3-isopropyliden-oxindol und 3-[4-(3-Methyl-benzamido)piperidino] propylchlorid | 13 | 182-184 (Diethylether) |
| d) | 5-<3-[4-(2-Methoxy-benzamido)piperidino] propoxy>-3-isopropyliden-oxindol<br>aus 5-Hydroxy-3-isopropyliden-oxindol und 3-[4-(2-Methoxy-benzamido)piperidino] propylchlorid | 18 | 172 (3-Propanol) |

Beispiel 2 (Fortsetzung)

| | Bezeichnung | Ausbeute % | Schmelzpunkt °C (Loesungsmittel) |
|---|---|---|---|
| e) | 6-[3-(4-Benzamido-piperidino)propoxy]-3-isopropyliden-cumaranon-2 aus 6-Hydroxy-3-isopropyliden-cumaranon-2 und 3-(4-Benzamido-piperidino)propylchlorid | 42 | 167 (Diethylether) |
| f) | 6-[3-(4-Cyclopropancarboxamido-piperidino)propoxy]-2-isopropyliden-cumaranon-3 aus 6-Hydroxy-2-isopropyliden-cumaranon-3 und 3-(4-Cyclopropancarboxamido-piperidino)propylchlorid | 45 | 165-167 (Diethylether) |
| g) | 6-[3-(4-Benzamido-piperidino)propoxy]-2-isopropyliden-cumaranon-3 aus 6-Hydroxy-2-isopropyliden-cumaranon-3 und 3-(4-Benzamido-piperidino)propylchlorid | 70 | 187-189 (2-Propanol) |
| h) | 4-<3-[4-(4-Fluor-benzamido)piperidino]propoxy>-2-isopropyliden-cumaranon-3 aus 4-Hydroxy-2-isopropyliden-cumaranon-3 und 3-[4-(4-Fluor-benzamido)piperidino]propylchlorid | 54 | 194 (Methanol) |

Beispiel 2 (Fortsetzung)

| | Bezeichnung | Ausbeute % | Schmelzpunkt°C (Loesungsmittel) |
|---|---|---|---|
| i) | 6-<3-[4-(4-Fluor-benzamido)piperidino] propoxy>-2-isopropyliden-cumaranon-3<br><br>aus 6-Hydroxy-2-isopropyliden-cumaranon-3 und 3-[4-(4-Fluor-benzamido)piperidino] propylchlorid | 56 | 175 (Methanol/ Diethylether) |
| j) | 6-<3-[4-(2-Nitro-benzamido)piperidino] propoxy>-2-isopropyliden-cumaranon-3<br><br>aus 6-Hydroxy-2-isopropyliden-cumaranon-3 und 3-[4-(2-Nitro-benzamido)piperidino] propylmesylat | 26 | 169 (Diethylether) |
| k) | 6-<3-[4-(2-Methylthio-benzamido)piperidino] propoxy>-2-isopropyliden-cumaranon-3<br><br>aus 6-Hydroxy-2-isopropyliden-cumaranon-3 und 3-[4-(2-Methylthio-benzamido)piperi-dino]propylmesylat | 20 | 140 (Diethylether) |
| l) | 6-<3-[4-(2-Methansulfonyl-benzamido) piperidino]propoxy>-2-isopropyliden-cumaranon-3<br>aus 6-Hydroxy-2-isopropyliden-cumaranon-3 und 3-[4-(2-Methansulfonyl-benzamido) piperidino]propylmesylat | 29 | 160 (Ethylacetat) |

Beispiel 2 (Fortsetzung)

| | Bezeichnung | Ausbeute % | Schmelzpunkt °C (Loesungsmittel) |
|---|---|---|---|
| m) | 6-<3-[4-(2-Nitro-phenylacetamido)piperidino]propoxy>-2-isopropyliden-cumaranon-3<br><br>aus 6-Hydroxy-2-isopropyliden-cumaranon-3 und 3-[4-(2-Nitro-phenylacetamido)piperidino]propylmesylat | 18 | Hydrochlorid<br>80-85<br>(Ethylacetat) |
| n) | 2-Isopropyliden-6-[3-(4-phenylacetamido-piperidino)propoxy]-7-propyl-cumaranon-3<br><br>aus 6-Hydroxy-2-isopropyliden-7-propyl-cumaranon-3 und 3-(4-Phenylacetamido-piperidino)propylchlorid | 53 | 159-161<br>(Diethylether) |
| o) | 6-[3-(4-Benzamido-piperidino)propoxy]-2-(1-geranyl-2-propyliden)cumaranon-3<br><br>aus 2-(1-Geranyl-2-propyliden)-6-hydroxy-cumaranon-3 und 3-(4-Benzamido-piperidino)propylchlorid | 51 | amorph<br>(Ligroin) |
| p) | 6-[3-(4-Benzamido-piperidino)propoxy]-2-[4-(2,6,6-trimethyl-1-cyclohexen-1-yl)-3-buten-2-yliden]cumaranon-3<br><br>aus 6-Hydroxy-2-[4-(2,6,6-trimethyl-1-cyclohexen-1-yl)-3-buten-2-yliden]cumaranon-3 und 3-(4-Benzamido-piperidino)propylchlorid | 35 | amorph<br>(Ligroin) |

Beispiel 2 (Fortsetzung)

| | Bezeichnung | Ausbeute % | Schmelzpunkt°C (Loesungsmittel) |
|---|---|---|---|
| q) | 6-[3-(4-Benzamido-piperidino)propoxy]-2-(1-farnesyl-2-propyliden)cumaranon-3<br><br>aus 2-(1-Farnesyl-2-propyliden)-6-hydroxy-cumaranon-3 und 3-(4-Benzamido-piperidino)propylchlorid | 32 | Oxalat<br>80<br>(Ethylacetat) |
| r) | 6-[3-(4-Benzamido-piperidino)propoxy]-2-(2-methyl-1-propyliden)cumaranon-3<br><br>aus 6-Hydroxy-2-(2-methyl-1-propyliden)cumaranon-3 und 3-(4-Benzamido-piperidino)propylchlorid | 44 | 93<br>(2-Propanol) |
| s) | 6-[3-(4-Benzamido-piperidino)propoxy]-2-cinnamyliden-cumaranon-3<br>aus 2-Cinnamyliden-6-hydroxy-cumaranon-3 und 3-(4-Benzamido-piperidino)propylchlorid | 60 | 178-180<br>(Ethanol) |
| t) | 6-[3-(4-Benzamido-piperidino)propoxy]-2-benzyliden-cumaranon-3<br>aus 2-Benzyliden-6-hydroxy-cumaranon-3 und 3-(4-Benzamido-piperidino)propylchlorid | 78 | 214-216<br>(Methanol) |

Beispiel 2 (Fortsetzung)

| | Bezeichnung | Ausbeute % | Schmelzpunkt°C (Loesungsmittel) |
|---|---|---|---|
| u) | 2-Benzyliden-6-<3-[4-(4-fluor-benzamido) piperidino]propoxy>cumaranon-3 aus 2-Benzyliden-6-hydroxy-cumaranon-3 und 3-[4-(4-Fluor-benzamido)piperidino] propylchlorid | 48 | 183-185 (2-Propanol) |
| v) | 6-[3-(4-Phenylacetamido-piperidino)propoxy]-7-propyl-cumaranon-3 aus 6-Hydroxy-cumaranon-3 und 3-(4-Phenylacetamido-piperidino]propylchlorid | 49 | 145-146 (Diethylether) |

**Beispiel 3**

**6-<3-[4-(2-Amino-benzamido)piperidino]propoxy>-2-isopropyliden-cumaranon-3**

Eine Mischung aus 9.8 g (31 mmol) 6-(3-Brom-propoxy)-2-iso-propyliden-cumaranon-3, 6.9 g (31 mmol) 4-(2-Amino-benzamido) piperidin, 10.1 g (0.1 mol) Triethylamin und 100 ml Tetrahydrofuran wird 16 h am Rueckfluß erhitzt. Darauf engt man ein, nimmt den Rueckstand in Dichlormethan auf, waescht neutral, engt ein und chromatographiert an Kieselgel (Elutionsmittel: Dichlormethan/Methanol 9 : 1).

Man erhaelt nach Anreiben mit Diethylether 4.9 g der Titelverbindung (35 % d.Th.) vom Schmp. 139 - 140°C.

Das als Ausgangsstoff verwendete 6-(3-Brom-propoxy)-2-iso-propyliden-cumaranon-3 kann wie folgt hergestellt werden:

Zu der Loesung von 25.0 g (0.13 mol) 6-Hydroxy-2-isopropyliden-cumaranon-3 und 40 ml 1,3-Dibrompropan in 130 ml Butanon gibt man bei 75°C innerhalb 3 h 20.0 g (0.15 mol) Kaliumcarbonat, erwaermt 48 h zum Rueckfluß, filtriert, engt das Filtrat ein und verreibt den Rueckstand mit Ligroin.

Es verbleiben 29.8 g der gewuenschten Verbindung (74 % d.Th.) vom Schmp. 80 - 82°C.

**Beispiel 4**

In analoger Weise wie in Beispiel 3 beschrieben erhaelt man:

| | Bezeichnung | Ausbeute % | Schmelzpunkt°C (Loesungsmittel) |
|---|---|---|---|
| a) | 2-Isopropyliden-6-[3-(4-phenylacetamido-piperidino)propoxy]cumaranon-3 aus 6-(3-Brom-propoxy)-2-isopropyliden-cumaranon-3 und 4-Phenylacetamido-piperidin | 38 | 150 (2-Propanol) |
| b) | 6-[3-(4-Amino-piperidino)propoxy]-2-isopropyliden-cumaranon-3 aus 6-(3-Brom-propoxy)-2-isopropyliden-cumaranon-3 und 4-Amino-piperidin | 83 | Hydrochlorid amorph (Diethylether) |

**Beispiel 5**

**6-[3-(4-Benzamido-piperidino)propoxy]-2-isopropyl-cumaranon-3**

Eine Loesung von 5.0 g (11 mmol) 6-[3-(4-Benzamido-piperidino) propoxyl-2-isopropyliden-cumaranon-3 (Beispiel 2 g) in 200 ml Methanol wird bei Raumtemperatur und 1 bar Wasserstoffdruck ueber 0.5 g Platinoxid hydriert. Nach Aufnahme der berechneten Menge wird filtriert, eingeengt und der Rueckstand aus 2-Pro-panol umkristallisiert.

Man erhaelt 3.6 g Titelverbindung (75 % d.Th.) vom Schmp. 146-147° C.

**Beispiel 6**

In analoger Weise wie in Beispiel 5 beschrieben erhaelt man:

| | Bezeichnung | Ausbeute % | Schmelzpunkt°C (Loesungsmittel) |
|---|---|---|---|
| a) | 6-[3-(4-Benzamido-piperidino) propoxy]-2-(2-methyl-propyl) cumaranon-3<br><br>aus 6-[3-(4-Benzamido-piperidino) propoxy]-2-(2-methyl-1-propyliden) cumaranon-3 (Beispiel 2 r) | 69 | 133-135 (Diethylether) |
| b) | 6-[3-(4-Benzamido-piperidino) propoxy]-2-(3-phenyl-propyl) cumaranon-3<br><br>aus 6-[3-(4-Benzamido-piperidino) propoxy]-2-cinnamyliden-cumaranon-3 (Beispiel 2 s) | 41 | 120-122 (Diethylether) |
| c) | 6-[3-(4-Benzamido-piperidino) propoxy]-2-cyclohexyl-cumaranon-3<br>aus 6-[3-(4-Benzamido-piperidino) propoxy]-2-cyclohexyliden-cumaranon-3 (Beispiel 1) | 60 | 150 (Ethylacetat) |
| d) | 5-〈3-[4-(4-Fluor-benzamido) piperidino]-propoxy〉-3-isopropyl-oxindol<br>aus 5-〈3-[4-(4-Fluor-benzamido) piperidino]-propoxy〉-3-isopropyliden-oxindol (Beispiel 2 b) | 63 | Hydrochlorid 145 (Ethylacetat) |

**Beispiel 7**

**6-[3-(4-Cyclopropancarboxamido-piperidino)propoxy]-2-iso-propyliden-cumaranon-3**

Zu einer Mischung aus 6.6 g (20 mmol) 6-[3-(4-Amino-piperidino) propoxy]-2-isopropyliden-cumaranon-3 (Beispiel 4 b) und 8.4 g (0.1 mol) Natriumhydrogencarbonat in 150 ml Dichlormethan tropft man die Loesung von 3.65 g (25 mmol) Cyclopropancarbonsaeurechlorid in 50 ml Dichlormethan, ruehrt 5 h bei Raumtemperatur, waescht neutral, engt die organische Phase ein und chromatographiert den Rueckstand an Kieselgel (Elutionsmittel:. Dichlormethan/Methanol 9 : 1).

Man erhaelt 1.8 g Titelverbindung (23 % d.Th.), identisch mit der Verbindung von Beispiel 2 f.

**Beispiel 8**

**2-Isopropyliden-6-[3-(4-phenylacetamido-piperidino)propoxy]-7-propyl-cumaranon-3**

In eine Loesung von 9.0 g (20 mmol) 6-[3-(4-phenylacetamido-piperidino)propoxy]-7-propyl-cumaranon-3 (Beispiel 2 v) in 50 ml Ethanol und 10 ml Aceton leitet man bei 20°C Ammoniak bis zur Saettigung ein, erwaermt 3 h zum Rueckfluß, engt ein und chromatographiert an Kieselgel (Elutionsmittel: Dichlormethanol 95 : 5).

Man isoliert 4.1 g der Titelverbindung (42 % d.Th.), identisch mit der Verbindung von Beispiel 2 n.

**Beispiel 9**

Es wurden Tabletten hergestellt: Jede Tablette enthaelt 10 mg 6-[3-(4-Benzamido-piperidino)propoxy]-2-isopropyliden-cumaranon-3. Die Tabletten wurden gemaeß der folgenden Rezeptur hergestellt:

| | |
|---|---|
| 6-[3-(4-Benzamido-piperidino)propoxy]-2-isopropyliden-cumaranon-3 | 10 g |
| Lactose | 80 g |
| Staerke | 29 g |
| Magnesiumstearat | 1 g |

Vorstehende Verbindung wurde fein pulverisiert und mit Lactose und Staerke vermischt. Das Gemisch wurde in herkoemmlicher Weise granuliert. Magnesiumstearat wurde zu dem Granulat gegeben und das Gemisch zu 1000 Tabletten mit einem Einzelgewicht von 0.12 g verpreßt.

## Patentansprüche

1. Bicyclischer Phenolether der allgemeinen Formel I

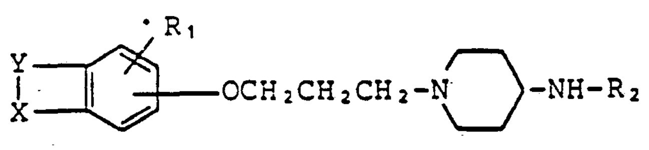

(I),

in welcher

$R_1$ Wasserstoff oder einen $C_1$-$C_6$-Alkylrest,

$R_2$ Wasserstoff, eine Phenacetyl-, $C_3$-$C_7$-Cycloalkyl-carbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkylsulfinyl, $C_1$-$C_6$-Alkylsulfonyl oder Cyano substituiert sein kann,

X ein Sauerstoffatom oder eine NH-Gruppe,

Y eine Gruppe $-CO-CHR_3-$, eine Gruppe $-CO-C=CR_4R_5$ oder $R_4R_5C=C-CO-$,

$R_3$ Wasserstoff, einen $C_1$-$C_6$-Alkylrest, der gegebenenfalls durch Phenyl substituiert sein kann oder einen $C_3$-$C_7$-Cycloalkylrest,

$R_4$ Wasserstoff oder einen $C_1$-$C_6$-Alkylrest und

$R_5$ Wasserstoff, einen $C_1$-$C_6$-Alkylrest, einen Phenyl- oder Styryl- oder einen Farnesyl-, Geranyl- und (2,6,6-Tri-methyl-1-cyclohexen-1-yl)-vinylrest bedeuten, wobei $R_4$ und $R_5$ zusammen mit dem C-Atom auch einen $C_3$-$C_7$-Cyclo-alkylring bilden können,

sowie deren Salze mit pharmakologisch verträglichen Säuren.

2. Verbindungen der allgemeinen Formel I gemäß Anspruch 1, in der $R_1$ Wasserstoff, $R_2$ eine Phenacetyl-, $C_3$-$C_7$-Cycloalkyl-carbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkylsulfinyl, $C_1$-$C_6$-Alkylsulfonyl oder Cyano substituiert sein kann, X eine NH-Gruppe und Y eine Gruppe $-CO-\underset{|}{C}=CR_4R_5$

darstellen, wobei $R_4$ und $R_5$ die angegebene Bedeutung haben.

3. Verbindungen der allgemeinen Formel I gemäß Anspruch 1, in der $R_1$ Wasserstoff oder eine n-Propylgruppe, $R_2$ eine Phenacetyl-, $C_3$-$C_7$-Cycloalkylcarbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkyl-sulfinyl, $C_1$-$C_6$-Alkylsulfonyl oder Cyano substituiert sein kann, X ein Sauerstoffatom und Y eine Gruppe $-CO-\underset{|}{C}=CR_4R_5$

darstellen, wobei $R_4$ und $R_5$ die angegebene Bedeutung haben.

4. Verbindungen der allgemeinen Formel I gemäß Anspruch 1, in der $R_1$ Wasserstoff oder eine n-Propylgruppe, $R_2$ eine Phenacetyl-, $C_3$-$C_7$-Cycloalkylcarbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkyl-sulfinyl, $C_1$-$C_6$-Alkylsulfonyl oder Cyano substituiert sein kann, X ein Sauerstoffatom und Y eine Gruppe -CO-$CHR_3$- darstellen, wobei $R_3$ die angegebene Bedeutung hat.

5. Verbindungen nach einem der vorhergehenden Ansprüche, in denen $R_2$ eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkylsulfinyl, $C_1$-$C_6$-Alkyl-sulfonyl oder Cyano substituiert sein kann oder eine Phenacetylgruppe bedeutet.

6. Verfahren zur Herstellung von bicyclischen Phenolethern der allgemeinen Formel I

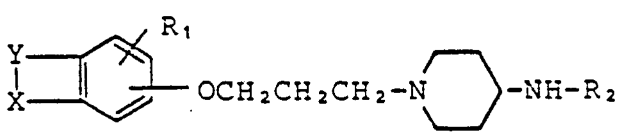

(I),

in welcher

$R_1$ Wasserstoff oder einen $C_1$-$C_6$-Alkylrest,

$R_2$ Wasserstoff, eine Phenacetyl-, $C_3$-$C_7$-Cycloalkyl-carbonyl- oder eine Benzoylgruppe, die gegebenenfalls durch Halogen, Hydroxyl, $C_1$-$C_6$-Alkyl, $C_1$-$C_6$-Alkoxy, Nitro, Amino, $C_1$-$C_6$-Alkylthio, $C_1$-$C_6$-Alkylsulfinyl, $C_1$-$C_6$-Alkylsulfonyl oder Cyano substituiert sein kann,

X ein Sauerstoffatom oder eine NH-Gruppe, Y eine Gruppe -CO-$CHR_3$-, eine Gruppe $-CO-\underset{|}{C}=CR_4R_5$ oder $R_4R_5C=\underset{|}{C}-CO-$,

$R_3$ Wasserstoff, einen $C_1$-$C_6$-Alkylrest, der gegebenenfalls durch Phenyl substituiert sein kann oder einen $C_3$-$C_7$-Cycloalkylrest,

$R_4$ Wasserstoff oder einen $C_1$-$C_6$Alkylrest und

$R_5$ Wasserstoff, einen $C_1$-$C_6$-Alkylrest, einen Phenyl- oder Styryl- oder einen Farnesyl-, Geranyl- und (2,6,6-Tri-methyl-1-cyclohexen-1-yl)-vinylrest bedeuten, wobei $R_4$ und $R_5$ zusammen mit dem C-Atom auch einen $C_3$-$C_7$-Cyclo-alkylring bilden können,

sowie deren Salze mit pharmakologisch verträglichen Säuren,

dadurch gekennzeichnet, daß man in an sich bekannter Weise eine Verbindung der allgemeinen Formel II

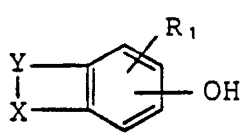

(II),

in der

$R_1$, X und Y die obengenannte Bedeutung haben, mit einer Verbindungen der allgemeinen Formel III

L-$CH_2CH_2CH_2$-M (III),

in der

L und M reaktive Reste bedeuten,

und einer Verbindung der allgemeinen Formel IV

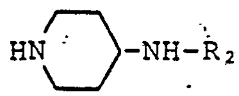

(IV),

in der
$R_2$ die obengenannte Bedeutung hat,
umsetzt,
anschließend gewuenschtenfalls die Gruppe $R_2$ durch bekannte Verfahren in eine andere Gruppe $R_2$ umwandelt,
fuer den Fall, daß $R_3$ ein Wasserstoffatom bedeutet, gegebenenfalls mit einer Verbindung $R_4COR_5$ oder einem reaktiven Derivat davon kondensiert,
fuer den Fall daß Y die Gruppe $-CO-\underset{|}{C}=CR_4R_5$
bedeutet, gewuenschtenfalls die Doppelbindung reduziert,
und das so erhaltene Reaktionsprodukt gewuenschtenfalls in ein pharmakologisch vertraegliches Salz ueberfuehrt.

7. Verwendung von Verbindungen gemäß Anspruch 1 zur Herstellung von Arzneimitteln mit antiallergischer Wirkung.

8. Arzneimittel, gekennzeichnet durch einen Gehalt an Verbindungen gemäß Anspruch 1.

**Claims**

1. Bicyclic phenol ethers of the general formula I

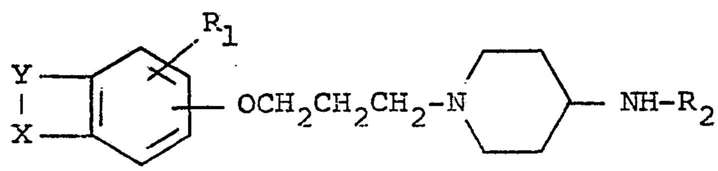

(I)

in which $R_1$ signifies hydrogen or a $C_1$-$C_6$ alkyl radical, $R_2$ hydrogen, a phenacetyl, $C_3$-$C_7$-cycloalkylcarbonyl or a benzoyl group, which can optionally be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, X an oxygen atom or an -NHgroup, Y a group -CO-CHR$_3$-, a group $-CO-\underset{|}{C}=CR_4R_5$ or $R_4R_5C=\underset{|}{C}-CO-$,
$R_3$ hydrogen, a $C_1$-$C_6$-alkyl radical, which can optionally be substituted by phenyl, or a $C_3$-$C_7$-cycloalkyl radical, $R_4$ hydrogen or a $C_1$-$C_6$-alkyl radical and $R_5$ hydrogen, a $C_1$-$C_6$-alkyl radical, a phenyl or styryl or a farnesyl, geranyl and (2,6,6-trimethyl-1-cyclohexen-1-yl)-vinyl radical, whereby $R_4$ and $R_5$ together with the C-atom can also form a $C_3$-$C_7$-cycloalkyl ring, as well as their salts with pharmacologically acceptable acids.

2. Compounds of the general formula I according to claim 1, in which $R_1$ represents hydrogen, $R_2$ a phenacetyl, $C_3$-$C_7$-cycloalkylcarbonyl or a benzoyl group which can possibly be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, X an NH group and Y a group $-CO-\underset{|}{C}=CR_4R_5$,
whereby $R_4$ and $R_5$ have the given meaning.

3. Compounds of the general formula I according to claim 1, in which $R_1$ represents hydrogen or an n-propyl group, $R_2$ a phenacetyl, $C_3$-$C_7$-cycloalkylcarbonyl or a benzoyl group, which can possibly be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, X an oxygen atom and Y a group $-CO-\underset{|}{C}=CR_4R_5$,
whereby $R_4$ and $R_5$ have the given meaning.

4. Compounds of the general formula I according to claim 1, in which $R_1$ represents hydrogen or an n-propyl group, $R_2$ a phenacetyl, $C_3$-$C_7$-cycloalkylcarbonyl or a benzoyl group, which can possibly be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, X an oxygen atom and Y a group -CO-CHR$_3$-, whereby $R_3$ has the given meaning.

5. Compounds according to one of the preceding claims, in which $R_2$ signifies a benzoyl group, which can possibly be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, or a phenacetyl group.

6. Process for the preparation of bicyclic phenol ethers of the general formula I

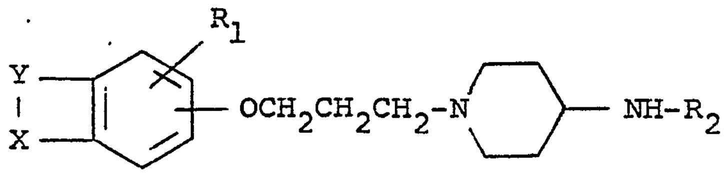 (I)

in which $R_1$ signifies hydrogen or a $C_1$-$C_6$-alkyl radical, $R_2$ hydrogen, a phenacetyl, $C_3$-$C_7$-cycloalkylcarbonyl or a benzoyl group, which can optionally be substituted by halogen, hydroxyl, $C_1$-$C_6$-alkyl, $C_1$-$C_6$-alkoxy, nitro, amino, $C_1$-$C_6$-alkylthio, $C_1$-$C_6$-alkylsulphinyl, $C_1$-$C_6$-alkylsulphonyl or cyano, X an oxygen atom or an -NHgroup, Y is a group -CO-CHR$_3$-, a group $-CO-C=CR_4R_5$ or $R_4R_5C=C-CO-$ ,

$R_3$ hydrogen, a $C_1$-$C_6$-alkyl radical, which can optionally be substituted by phenyl, or a $C_3$-$C_7$-cycloalkyl radical, $R_4$ hydrogen or a $C_1$-$C_6$-alkyl radical and $R_5$ hydrogen, a $C_1$-$C_6$-alkyl radical, a phenyl or styryl or a farnesyl, geranyl and (2,6,6-trimethyl-1-cyclohexen-1-yl)-vinyl radical, whereby $R_4$ and $R_5$ together with the C-atom can also form a $C_3$-$C_7$-cyclo-alkyl ring, as well as their salts with pharmacologically acceptable acids, characterised in that, in per se known manner, one reacts a compound of the general formula II

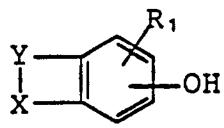 (II)

in which $R_1$, X and Y have the above-given meaning, with a compound of the general formula III
$L-CH_2CH_2CH_2-M$ (III)
in which L and M signify reactive residues, and a compound of the general formula IV

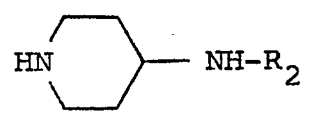 (IV)

in which $R_2$ has the above-given meaning, subsequently, if desired, converts the group $R_2$ by known processes into another group $R_2$, for the case in which $R_3$ is a hydrogen atom optionally condenses with a compound $R_4$.C0.$R_5$ or a reactive derivative thereof, for the case in which Y signifies the group $-CO-C=CR_4R_5$, if

if desired reduces the double bond, and, if desired, converts the so obtained reaction product into a pharmacologically acceptable salt.

7. Use of compounds according to claim 1 for the preparation of medicaments with anti-allergic action.

8. Medicaments, characterised by a content of compounds according to claim 1.

**Revendications**

1- Ether bicyclique de phénol de formule générale I

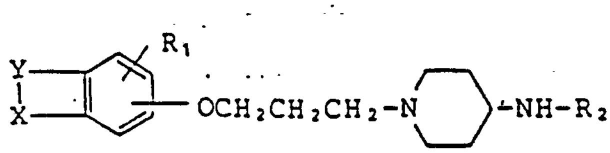 (I),

dans laquelle
$R_1$ représente l'hydrogègne ou un radical alkyle en $C_1$ à $C_6$, $R_2$ l'hydrogène, un groupe phénacétyle, cycloalkylcarbonyle en $C_3$ à $C_7$ ou benzoyle, qui peut éventuellement être substitué par un halogène, un groupe hydroxyle, alkyle en $C_1$ à $C_6$, alcoxyle en $C_1$ à $C_6$, nitro, amino, alkylthio en $C_1$ à $C_6$ alkylsulfinyl en $C_1$ à $C_6$, alkylsulfonyl en $C_1$ à $C_6$ ou cyano, X, un atome d'oxygène ou un groupe NH, Y, un groupe -CO-CHR$_3$-, un groupe $-CO-C=CR_4R_5$ ou $R_4R_5C=C-CO-$, R3,

$R_3$, l'hydrogène, un radical alkyle en $C_1$ à $C_6$ qui peut éventuellement être substitué par un groupe phényle, ou un radical cycloalkyle en $C_3$ à $C_7$, $R_4$ l'hydrogène ou un radical alkyle en $C_1$ à $C_6$ et $R_5$ l'hydrogène, un radical alkyle en $C_1$ à $C_6$, un radical phényle ou styryle ou un radical farnésyle, géranyle et (2,6,6-triméthyl-1-cyclohexène-1-yl)-vinyle, $R_4$ et $R_5$ pouvant aussi former, avec l'atome C, un noyau cycloalkyle en $C_3$ à $C_7$, ainsi que leurs sels d'acides pharmacologiquement tolérés.

2- Composés de formule générale I selon la revendication 1, dans laquelle $R_1$ représente l'hydrogène, $R_2$ un groupe phénacétyle, cycloalkylcarbonyle en $C_3$ à $C_7$ ou un groupe benzoyle, qui peut éventuellement être substitué par un halogène, un groupe hydroxyle, alkyle en $C_1$ à $C_6$, alcoxyle en $C_1$ à $C_6$, nitro, amino, alkylthio en $C_1$ à $C_6$, alkylsulfinyle en $C_1$ à $C_6$, alkylsulfonyle en $C_1$ à $C_6$ ou cyano, X un groupe NH et Y un groupe $-CO-C=CR_4R_5$,
$R_4$ et $R_5$ ayant la signification indiquée.

3- Composés de formule générale I selon la revendication 1 dans laquelle $R_1$ représente l'hydrogène ou un groupe n-propyle, $R_2$ un groupe phénacétyle, cycloalkylcarbonyle en $C_3$ à $C_7$ ou un groupe benzoyle, qui peut éventuellement être substitué par un halogène, un groupe hydroxyle, alkyle en $C_1$ à $C_6$. alcoxyle en $C_1$ à $C_6$, nitro, amino, alkylthio en $C_1$ à $C_6$, alkylsulfinyle en $C_1$ à $C_6$, alkylsulfonyle en $C_1$ à $C_6$, ou cyano, X un atome d'oxygène et Y un groupe $-CO-C=CR_4R_5$,
$R_4$ et $R_5$ ayant la signification indiquée.

4- Composés de formule générale I selon la revendication 1, dans laquelle $R_1$ représente l'hydrogène ou un groupe n-propyle, $R_2$ un groupe phénacétyle, cycloalkylcarbonyle en $C_3$ à $C_7$ ou un groupe benzoyle, qui peut éventuellement être substitué par un halogène, un groupe hydroxyle, alkyle en $C_1$ à $C_6$, alcoxyle en $C_1$ à $C_6$, nitro, amino, alkylthio en $C_1$ à $C_6$, alkylsulfinyle en $C_1$ à $C_6$, alkylsulfonyle en $C_1$ à $C_6$ ou cyano, X un atome d'oxygène et Y un groupe $-CO-CHR_3$, $R_3$ ayant la signification indiquée.

5- Composés selon l'une des revendications précédentes dans lesquels $R_2$ représente un groupe benzoyle, qui peut éventuellement être substitué par un halogène, un groupe hydroxyle, alkyle en $C_1$ à $C_6$, alcoxyle en $C_1$ à $C_6$, nitro, amino, alkylthio en $C_1$ à $C_6$, alkylsulfinyle en $C_1$ à $C_6$, alkylsulfonyle en $C_1$ à $C_6$ ou cyano, ou représente un groupe phénacétyle.

6- Procédé pour la préparation d'éthers bicycliques de phénol de formule générale I

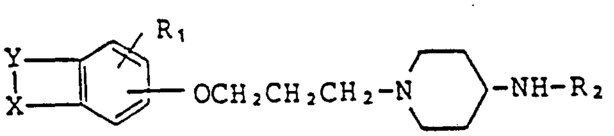 (I),

dans laquelle

$R_1$ représente l'hydrogène ou un radical alkyle en $C_1$ à $C_6$, $R_2$ l'hydrogène, un groupe phénacétyle, cycloalkylcarbonyle en $C_3$ à $C_7$ ou benzoyle, qui peut éventuellement être substitué par un halogène, un groupe hydroxyle, alkyle en $C_1$ à $C_6$, alcoxyle en $C_1$ à $C_6$, nitro, amino, alkylthio en $C_1$ à $C_6$ alkylsulfinyl en $C_1$ à $C_6$, alkylsulfonyl en $C_1$ à $C_6$ ou cyano, X, un atome d'oxygène ou un groupe NH, Y, un groupe $-CO-CHR_3-$, un groupe $-CO-C=CR_4R_5$ ou $R_4R_5C=C-CO-$,

$R_3$, l'hydrogène, un radical alkyle en $C_1$ à $C_6$ qui peut éventuellement être substitué par un groupe phényle, ou un radical cycloalkyle en $C_3$ à $C_7$, $R_4$ l'hydrogène ou un radical en $C_1$ à $C_6$ et $R_5$ l'hydrogène, un radical alkyle en $C_1$ à $C_6$ un radical phényle ou styryle ou un radical farnésyle, géranyle et (2,6,6-trimèthyl-1-cyclohexène-1-yl)-vinyle, $R_4$ et $R_5$ pouvant aussi former, avec l'atome C, un noyau cycloalkyle en $C_3$ à $C_7$, ainsi que leurs sels d'acides pharmacologiquement tolérés, caractérisé par le fait que de manière en elle-même connue, on fait réagir un composé de la formule générale II

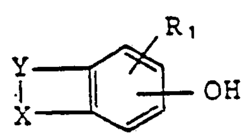 (II),

dans laquelle $R_1$, X et Y ont la signification indiquée ci-dessus, sur un composé de formule générale III
$L-CH_2CH_2CH_2-M$ (III)
dans laquelle L et M représentent des radicaux réactifs, et un composé de formule générale IV

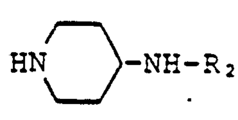 (IV),

dans laquelle $R_2$ a la signification indiquée ci-dessus, qu'ensuite si on le désire, on convertit le groupe $R_2$ par des procédés connus en un autre groupe $R_2$, que dans le cas où $R_3$ représente un atome d'hydrogène, on condense éventuellement avec un composé $R_4COR_5$, ou un dérivé réactif de celui-ci, que dans le cas où Y représente le groupe $-CO-\underset{|}{C}=CR_4R_5$,

on réduit si on le désire la double liaison, et que si on le désire, on convertit le produit de réaction ainsi obtenu en un sel pharmacologiquement toléré.

7- Utilisation de composés selon la revendication 1 pour la préparation de médicaments à action antiallergique,

8- Médicaments caractérisés par une teneur en composés selon la revendication 1.